# FASCICULE DE BREVET EUROPEEN

(11) **EP 2 901 162 B1**
(45) Date de publication et mention de la délivrance du brevet: **22.05.2019**
(21) Numéro de dépôt: 13786523.4
(22) Date de dépôt: 26.09.2013
(51) Int. Cl.: G01N 33/68

(54) **PROCEDE POUR LE DIAGNOSTIC DE DYSTROPHIES MUSCULAIRES**
VERFAHREN ZUR DIAGNOSE VON MUSKELDYSTROPHIE
METHOD FOR DIAGNOSING MUSCULAR DYSTROPHY

(30) Priorité: 26.09.2012 FR 1259023
(43) Date de publication de la demande: 05.08.2015
(73) Titulaire: Genethon, 91000 Evry (FR)
(72) Inventeur: ROUILLON, Jérémy, F-91100 Corbeil-Essonnes (FR); SVINARTCHOUK, Fedor, F-94800 Villejuif (FR)
(74) Mandataire: Sekhri, Redha
(86) Numéro de dépôt international: PCT/FR2013/052280
(87) Numéro de publication internationale: WO 2014/049282

(56) Documents cités:
- EP-A1- 2 407 784
- EFSTATHIOS VASSILIADIS ET AL: "Clinical evaluation of a matrix metalloproteinase-12 cleaved fragment of titin as a cardiovascular serological biomarker", JOURNAL OF TRANSLATIONAL MEDICINE, BIOMED CENTRAL, LONDON, GB, vol. 10, no. 1, 6 juillet 2012 (2012-07-06), page 140, XP021121805, ISSN: 1479-5876, DOI: 10.1186/1479-5876-10-140
- MATSUMURA K ET AL: "Immunochemical study of connectin (titin) in neuromuscular diseases using a monoclonal antibody: connectin is degraded extensively in Duchenne muscular dystrophy", JOURNAL OF NEUROLOGICAL SCIENCES, ELSEVIER SCIENTIFIC PUBLISHING CO, AMSTERDAM, NL, vol. 93, no. 2-3, 1 novembre 1989 (1989-11-01), pages 147-156, XP024302381, ISSN: 0022-510X, DOI: 10.1016/0022-510X(89)90185-8 [extrait le 1989-11-01]
- NADARAJAH V D ET AL: "Serum matrix metalloproteinase-9 (MMP-9) as a biomarker for monitoring disease progression in Duchenne muscular dystrophy (DMD)", NEUROMUSCULAR DISORDERS, PERGAMON PRESS, GB, vol. 21, no. 8, 27 mai 2011 (2011-05-27), pages 569-578, XP028252104, ISSN: 0960-8966, DOI: 10.1016/J.NMD.2011.05.011 [extrait le 2011-06-04]
- EFSTATHIOS VASSILIADIS ET AL: "Clinical evaluation of a matrix metalloproteinase-12 cleaved fragment of titin as a cardiovascular serological biomarker", JOURNAL OF TRANSLATIONAL MEDICINE, BIOMED CENTRAL, vol. 10, no. 1, 6 July 2012 (2012-07-06), page 140, XP021121805, ISSN: 1479-5876, DOI: 10.1186/1479-5876-10-140

## Description

### DOMAINE DE L'INVENTION

L'invention concerne un procédé de diagnostic, de pronostic et de suivi thérapeutique d'une dystrophie musculaire, par détection de la titin, ou d'un ou plusieurs fragments de la titin dans dans un liquide corporel d'un sujet, de préférence dans l'urine ou dans le sang.

### ETAT DE LA TECHNIQUE

Les dystrophies musculaires de Duchenne (DMD) ou de Becker (BMD) sont causées par des mutations ou des délétions du gène codant la dystrophine (Muntoni, Torelli et al. 2003). Dans le premier cas, où le phénotype est le plus sévère, la dystrophine est totalement absente. Le complexe DAPC (Dystrophine Associated Protein Complex), qui permet de relier les filaments intracellulaires d'actine à la matrice extracellulaire (Le Rumeur, Winder et al.), est également manquant. Ce complexe protège habituellement la membrane des fibres musculaires qui sont soumises aux contractions et aux relâchements. En son absence, les fibres ne sont plus protégées, on observe dans les muscles des cellules musculaires en dégénérescence et des nouvelles cellules qui témoignent d'une régénération tendant à contrebalancer le phénomène (Batchelor and Winder 2006). A terme, la régénération est insuffisante et les fibres sont remplacées par du tissus adipeux.

Sur le plan thérapeutique, de grands espoirs reposent actuellement sur la technique du saut d'exon (Cirak, Arechavala-Gomeza et al.; Lu, Yokota et al.). En effet, la BMD, qui mène à un phénotype moins grave, est également due à une ou plusieurs mutations dans le gène codant pour la dystrophine mais les domaines fondamentaux de la protéine sont conservés :
- un domaine N-terminal de liaison aux filaments d'actine,
- un domaine C-terminal riche en cystéines qui se lie au complexe DAPC.

Pour les patients DMD, il est donc possible d'obtenir un phénotype Becker en excluant les exons porteurs de mutations non-sens au sein des ARN messagers (ARNm) et ainsi rétablir le cadre de lecture ouvert. La protéine produite, plus courte, est alors partiellement fonctionnelle. Cette stratégie est actuellement testée via plusieurs essais cliniques.

Une surveillance médicale régulière, pluridisciplinaire, permet d'évaluer l'évolution de la pathologie et de proposer une prise en charge permettant d'améliorer la vie des patients. Il s'agit de prévention des rétractions, d'apport d'aides techniques, kinésithérapie, surveillance cardiaque, orthopédie et aide respiratoire. Le suivi diagnostique est réalisé entre autre par l'évaluation des fonctions motrices, par des biopsies musculaires ou par le dosage d'une enzyme sécrétée dans la circulation, la créatine kinase (Bushby, Finkel et al.).

L'analyse de biopsie musculaire permet d'observer les fibres lésées, des fibres plus petites témoignant de la régénération musculaire, ainsi que des zones de nécrose remplacées par du tissu adipeux. Cette méthode a pour inconvénient d'être très invasive pour le patient.

Une autre méthode consiste à doser la créatine kinase (CK) dans le sang. Cette enzyme est liée au métabolisme énergétique présent dans plusieurs types de cellules. L'augmentation de sa concentration dans le sang témoigne de l'état de dégradation des fibres musculaires. Cependant, ce biomarqueur n'est pas totalement fiable car son niveau dépend également de stress comme l'activité physique (Nicholson, Morgan et al. 1986). Par ailleurs, sa détection nécessite des méthodes invasives (prises de sang). Son niveau est généralement mesuré après l'apparition des premiers signes cliniques aux alentours de 3 ans. La dégradation progressive des muscles chez les patients DMD diminue la fiabilité de ce marqueur avec l'âge.

Par conséquent, il apparait nécessaire d'identifier de nouveaux biomarqueurs plus fiables pour l'identification d'une dystrophie musculaire, marqueurs qui pourraient être dosés à partir de prélèvements non invasifs ou peu invasifs, comme le prélèvement d'urine, de sang, de sérum ou de plasma.

Vassiliadis et al. décrivent la détection de la titin dans les sérums de patients atteints de maladies cardiovasculaires.

Matsumura et al. décrivent la dégradation de la titin dans les muscles de patients atteints d'une dystrophie musculaire de duchenne.

Les inventeurs ont pu identifier la présence d'une protéine particulière dans les liquides corporels de patients atteints d'une dystrophie musculaire et absente dans les liquides corporels de sujets sains. L'exploitation de cette avancée majeure fait l'objet de la présente divulgation.

### RESUME DE L'INVENTION

Les inventeurs ont étudié des échantillons de liquides corporels de patients atteints de dystrophies musculaires afin de déterminer si des biomarqueurs spécifiques de ces maladies pouvaient être identifiés. Ces travaux ont permis de mettre en évidence la présence de la titin chez les patients malades par rapport aux donneurs sains.

La constatation de la présence de ce marqueur chez un individu malade par rapport à un individu sain trouve une application dans le domaine du diagnostic. La présente divulgation se rapporte ainsi à l'utilisation de la titin ou d'un ou plusieurs fragments de cette dernière, pour la mise en oeuvre d'un procédé de diagnostic d'une dystrophie musculaire, en particulier de la dystrophie musculaire de Duchenne. Selon la présente divulgation, le procédé pour le diagnostic, le pronostic ou le suivi de l'efficacité d'un traitement d'une dystrophie musculaire chez un sujet comprend notamment la détection de la présence ou l'absence de la titin ou d'un ou plusieurs fragments de la titin, dans un échantillon de liquide corporel dudit sujet. Selon la présente divulgation, la présence de la titin ou d'un fragment de la titin est indicative d'une dystrophie musculaire, en particulier de la dystrophie musculaire de Duchenne.

L'invention concerne un procédé pour le diagnostic d'une dystrophie musculaire, comprenant la détermination dans un échantillon biologique d'un sujet de la quantité de titin ou d'un ou plusieurs fragments de celle-ci, ledit échantillon biologique étant un échantillon d'urine.

Un niveau plus élevé de titin ou d'un ou plusieurs fragments de la titin dans l'échantillon prélevé du sujet testé par rapport à un sujet sain sera indicatif d'une dystrophie musculaire.

La présence de la titin ou d'un ou plusieurs fragments de celle-ci dans un liquide corporel de patients atteints d'une dystrophie musculaire, et en particulier de la DMD, n'a jamais été rapportée par des publications antérieures.

La présente divulgation concerne également un procédé de suivi de l'évolution d'une dystrophie musculaire (ou procédé de pronostic). La présente invention concerne également un procédé pour l'évaluation de l'efficacité d'un traitement thérapeutique d'une dystrophie musculaire. Dans ce cas, le procédé comprend la détection de la titin ou d'un ou plusieurs fragments de celle-ci dans un second échantillon de liquide corporel d'un sujet, ce niveau dans l'échantillon du sujet étant comparé au niveau de ladite titin ou desdits un ou plusieurs fragments dans un premier échantillon de référence qui correspond à un échantillon prélevé antérieurement sur le même sujet. Dans le cas du suivi de l'efficacité d'un traitement, le premier échantillon pourra avoir été prélevé avant l'administration du traitement thérapeutique au sujet, et le second échantillon sera prélevé après administration du traitement thérapeutique (par exemple plusieurs jours/semaines/mois après administration du traitement thérapeutique). Alternativement, les premier et second échantillons peuvent être prélevés tous les deux après administration du traitement thérapeutique (par exemple, le premier échantillon est prélevé après traitement, le même jour que ce traitement, ou plusieurs jours/semaines/mois après le traitement, et le second échantillon est prélevé plusieurs jours/semaines/mois après le premier échantillon). Alternativement, les échantillons peuvent être comparés aux échantillons dits « standard » d'autres patients ayant la même maladie.

L'invention concerne par ailleurs une trousse utile pour le diagnostic d'une dystrophic musculaire.

### DESCRIPTION DETAILLEE DE L'INVENTION

Dans le cadre de l'invention, un "échantillon de référence", ou encore "échantillon standard" lorsqu'il est fait mention d'un "échantillon sain", correspond à un échantillon de liquide corporel obtenu à partir d'un ou plusieurs sujets, de préférence deux ou plus, qui ne souffrent pas de dystrophie musculaire. L'échantillon de référence peut également correspondre à un échantillon obtenu à partir d'un ou plusieurs patients souffrant d'une dystrophie musculaire. Dans le cadre de méthodes pour le suivi d'une dystrophie musculaire ou pour le suivi de l'efficacité d'un traitement, l'échantillon de référence peut notamment être un échantillon prélevé sur le sujet qui sera suivi, mais avant que le suivi ait débuté.

Par "dystrophie musculaire", on désigne notamment la dystrophie musculaire de Duchenne, la dystrophie musculaire de Becker, les dystrophies musculaires des ceintures comme, par exemple, les alpha- et gamma-sarcoglycanopathies. L'invention se rapporte plus particulièrement à l'étude d'une dystrophie musculaire de Duchenne.

Selon l'invention, l'échantillon biologique du sujet étudié est un échantillon d'urine.

On entend par "sujet" un mammifère, humain ou non humain, de préférence humain. Le sujet peut présenter une prédisposition pour une dystrophie musculaire (révélée par exemple par analyse génétique, ou une suspicion pouvant résulter d'antécédents familiaux) ou souffrir d'une dystrophie musculaire déclarée. L'invention peut également être appliquée en dépistage, le sujet ne présentant aucun symptôme ou prédisposition connue. En particulier, le procédé selon l'invention peut être appliqué au dépistage de masse chez les jeunes enfants ou bien chez les nouveau-nés. L'invention peut également être appliquée au diagnostic prénatal d'une dystrophie musculaire. L'invention peut également être appliquée au suivi des modèles animaux, y compris les modèles canins, notamment au chien GRMD (Golden Retriever Muscular Dystrophy), et murins (notamment souris mdx) lors de la mise au point préclinique de traitements.

Les inventeurs ont notamment montré que la titin ou un ou plusieurs fragments de cette dernière est particulièrement utile pour la détection ou le suivi d'une dystrophie dans la petite enfance puisqu'il est détectable au moins dès l'âge de 3 ans, et probablement avant. Ainsi, selon un mode particulier de réalisation, le sujet est un adulte, un adolescent ou un enfant, notamment un enfant âgé de moins de 10 ans, de moins de 5 ans, de moins de 4 ans, ou le sujet est un enfant de 3 ans ou de moins de 3 ans, ou le sujet est un nouveau-né. Il en résulte que la détection de la maladie pourra se faire à un stade très précoce.

La présente divulgation vise également un procédé de diagnostic d'une dystrophie musculaire, comprenant la détection de la présence ou de l'absence de la titin, ou d'un fragment de la titin, dans un échantillon de liquide corporel d'un sujet, la présence de ladite titin ou dudit fragment étant indicative d'une dystrophie musculaire.

L'invention vise notamment la détection de la titin dans un échantillon d'urine d'un sujet. A l'heure actuelle, il n'existe aucun moyen de diagnostic de dystrophies musculaires à partir d'urine.

Une approche qui peut permettre le diagnostic précoce de ces maladies sur un grand nombre de patients permettra d'identifier des candidats pour le diagnostic par séquençage. De même, plus tôt le dépistage peut être effectué, plus le traitement par thérapie génique sera efficace, palliant à la lyse musculaire et l'épuisement du stock de cellules musculaires.

Ainsi, selon un mode de réalisation, la présente invention concerne un procédé de diagnostic précoce d'une dystrophie musculaire, en particulier de la dystrophie musculaire de Duchenne.

La Titin (TITIN_HUMAN, UniProt Knowledgebase number : Q8WZ42) est la plus grande protéine (38138 résidus; PM 3.8 MDa) parmi les protéines codées par le génome humain et elle participe à l'organisation d'un sarcomère. Cette protéine est importante pour l'assemblage et la stabilité du sarcomère et son organisation possède différents types de modules et des zones de contacts multiples avec des protéines du sarcomère. Dans les muscles squelettiques et cardiaques de vertébrés, chaque molécule de titin adhère aux filaments épais de myosine sur presque toute sa longueur, et participe à l'élasticité de chaque demi-sarcomère de la ligne M à la ligne Z. Les mutations trouvées sur la titin sont responsables d'un grand nombre de pathologies musculaires : des cardiomyopathies dilatées (CMD1G) ; d'un type de Dystrophie des ceintures (LGMD 2J) ; et de l'EOMFC (early-onset myopathy with fatal cardiomyopathy). Onze isoformes de la titin produites par l'epissage alternatif sont bien identifiées de Q8WZ42-1 à Q8WZ42-11 :
Isoforme 1 (Identifiant: Q8WZ42-1; SEQ ID NO:1) - Séquence canonique;
Isoforme 2 (Identifiant: Q8WZ42-2; SEQ ID NO:2) - La séquence de cette isoforme diffère de la séquence canonique comme suit: 555-646: Manquant;
Isoforme 3 (Identifiant: Q8WZ42-3; SEQ ID NO:3) - Aussi connu comme: Small cardiac N2-B; La séquence de cette isoforme diffère de la séquence canonique comme suit: 556-601: Manquant; 4474-11851: Manquant;
Isoforme 4 (Identifiant: Q8WZ42-4; SEQ ID NO:4) - Aussi connu comme: Soleus; La séquence de cette isoforme diffère de la séquence canonique comme suit: 3454-4380: Manquant; 11507-11507: E → EVFEEPEESPSAPPKKPEVPPVR;
Isoforme 5 (Identifiant: Q8WZ42-5; SEQ ID NO:5) - La séquence de cette isoforme diffère de la séquence canonique comme suit: 10382-10645: Manquant; 10742-10931: Manquant; 11015-11163: Manquant; 11223-11852: Manquant; 11985-12201: Manquant.
Isoforme 6 (Identifiant: Q8WZ42-6; SEQ ID NO:6) - Aussi connu comme: Small cardiac novex-3. La séquence de cette isoforme diffère de la séquence canonique comme suit: 3455-5604: FSSSFLSAEE...VLDLIIPPSF → LFSEGESEHS...AESFAALTLT; 5605-34350: Manquant.
Isoforme 7 (Identifiant: Q8WZ42-7; SEQ ID NO:7) - Aussi connu comme: Cardiac novex-2. La séquence de cette isoforme diffère de la séquence canonique comme suit: 3435-3645: APESILHERI...LPAIFEYTVV → VQALDRQSSG...IEQEIEMEMK; 3646-4380: Manquant. Isoforme 8 (Identifiant: Q8WZ42-8; SEQ ID NO:8)- Aussi connu comme: Cardiac novex-1. La séquence de cette isoforme diffère de la séquence canonique comme suit: 3434-3434; E → EGFSKFEENT...CAATLTVTPK
Isoforme 9 (Identifiant: Q8WZ42-9; SEQ ID NO:9) - La séquence de cette isoforme diffère de la séquence canonique comme suit: 556-601: Manquant; 3434-3434: E → EVQALDRQSS...TTSAVLSVEG; 4474-11851: Manquant.
Isoform 10 (Identifiant: Q8WZ42-10; SEQ ID NO:10) - La séquence de cette isoforme diffère de la séquence canonique comme suit: 556-601: Manquant; 3434-3434: E → EGFSKFEENT...CAATLTVTPK; 4474-11851: Manquant.
Isoforme 11 (Identifiant: Q8WZ42-11; SEQ ID NO:11) - La séquence de cette isoforme diffère de la séquence canonique comme suit: 3454-4380: Manquant.
La portion centrale de la titin varie selon les isoformes mais les premiers 556 amino acides sont les mêmes dans ces isoformes.
La séquence de la titin est très conservée : il y a par exemple 89.4% d'homologie entre la protéine humaine et la protéine de la souris (TITIN_Mouse, UniProtKB Knowledgebase number : A2ASS6).

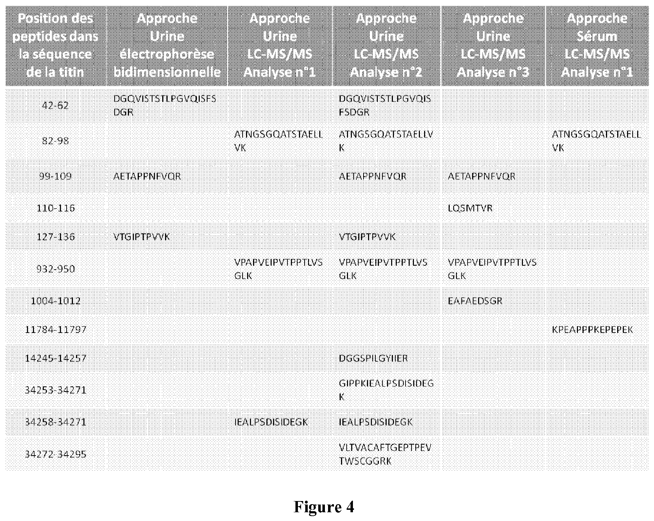

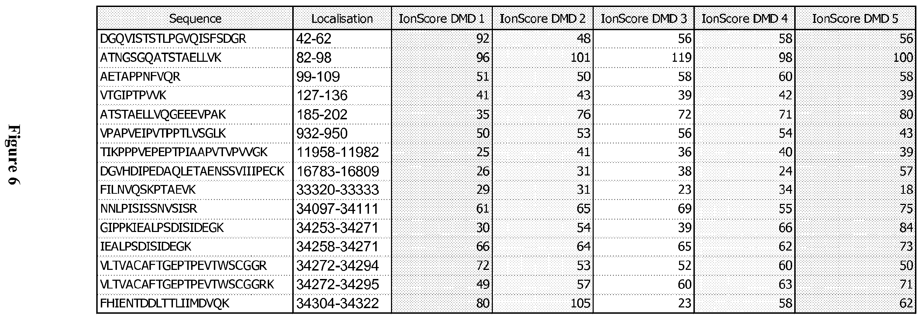

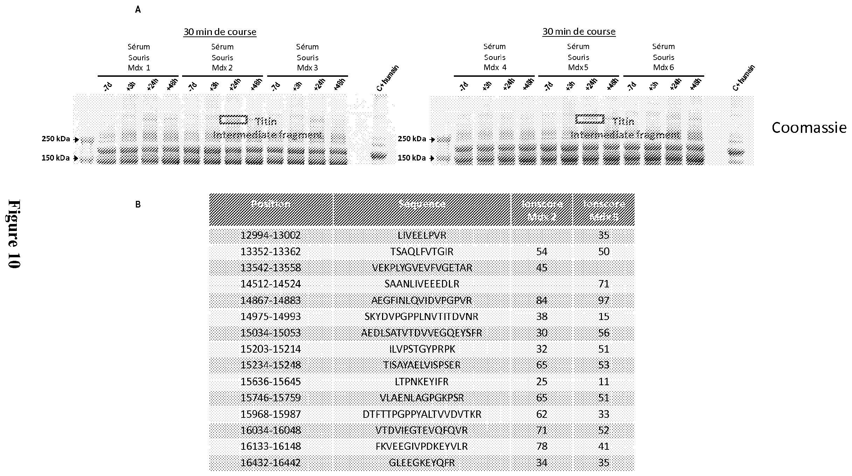

Selon un mode particulier de réalisation, les fragments de la titin détectés incluent des fragments contenant les peptides représentés dans le tableau de la figure 4 ou de la figure 6 ou de la figure 10,B. Ainsi, à titre illustratif, les fragments détectés selon l'invention comprennent des fragments de la titin comprenant les acides aminés 42-62 (SEQ ID NO:12), 82-98 (SEQ ID NO:13), 99-109 (SEQ ID NO:14), 110-116 (SEQ ID NO:15), 127-136 (SEQ ID NO:16), 932-950 (SEQ ID NO:17), 1004-1012 (SEQ ID NO:18), 11784-11797 (SEQ ID NO:19), 14245-14257 (SEQ ID NO:20) 34253-34271 (SEQ ID NO:21), 34258-34271 (SEQ ID NO:22), 34272-34295 (SEQ ID NO:23), 185-202 (SEQ ID NO:24), 11958-11982 (SEQ ID NO:25), 16783-16809 (SEQ ID NO:26), 33320-33333 (SEQ ID NO:27), 34097-34111 (SEQ ID NO:28), 34272-34294 (SEQ ID NO:29) et/ou 34304-34322 (SEQ ID NO:30). Selon un autre mode de réalisation, les fragments selon l'invention sont des fragments correspondant à la partie N-terminale de la titin, comprenant notamment les 300 premiers acides aminés de la protéine. Selon un autre mode de réalisation, les fragments selon l'invention sont des fragments correspondant à la partie C-terminale de la titin, comprenant notamment les 150 derniers acides aminés de la protéine. Selon un autre mode de réalisation, les fragments selon l'invention sont des fragments intermédiaires compris entre les parties N-terminale et C-terminale de la titin, notamment un fragment compris entre les acides aminés 12132 et 15880 de SEQ ID NO:1. Par exemple, le fragment détecté peut comprendre, dans la séquence de la titin chez la souris, les acides aminés 12994-13002 (SEQ ID NO:31), 13352-13362 (SEQ ID NO:32), 13542-13558 (SEQ ID NO:33), 14512-14524 (SEQ ID NO:34), 14867-14883 (SEQ ID NO:35), 14975-14993 (SEQ ID NO:36), 15034-15053 (SEQ ID NO:37), 15203-15214 (SEQ ID NO:38), 15234-15248 (SEQ ID NO:39), 15636-15645 (SEQ ID NO:40), 15746-15759 (SEQ ID NO:41), 15968-15987 (SEQ ID NO:42), 16034-16048 (SEQ ID NO:43), 16133-16148 (SEQ ID NO:44) et/ou 16432-16442 (SEQ ID NO:45). L'invention comprend également la détection de fragments de la titin correspondant dans la séquence de la titin humaine aux positions d'acides aminés des séquences SEQ ID NO:31 à SEQ ID NO:45.

Selon un mode particulier de réalisation, le procédé selon l'invention comprend la mesure du niveau d'au moins une des séquences SEQ ID NO:1 à SEQ ID NO:45, ou d'une séquence présentant une identité d'au moins 90 %, en particulier au moins 95 %, plus particulièrement d'au moins 98 % ou d'au moins 99 % à l'une des séquences SEQ ID NO:1 à SEQ ID NO:45.

La présence de titin ou d'un fragment de la titin peut être détectée par toute méthode conventionnelle, telle que
- ELISA (Enzyme-linked immunosorbent assay),
- Western-blotting,
- Spectrométrie de masse
- ou encore par toute autre méthode permettant la détection spécifique d'une protéine.

En particulier, les techniques d'ELISA et de western-blotting sont bien connues de l'homme du métier et mettent en oeuvre l'utilisation d'anticorps spécifiques de la protéine à détecter. Tout anticorps capable de fixer spécifiquement la titin, un ou des fragments de celle-ci, et plus particulièrement un fragment N-terminal ou C-terminal de la titin, comprenant notamment environ les 300 acides aminés N-terminaux ou les 150 acides aminés C-terminaux de la titin peut être utilisé dans le cadre de la présente invention. Ceci inclut les anticorps polyclonaux ou monoclonaux reconnaissant la titin ou un épitope de cette dernière, et en particulier des anticorps polyclonaux ou monoclonaux spécifiques de la partie N-terminale ou de la partie C-terminale de la titin. L'homme du métier est en mesure de produire des anticorps spécifiques d'une protéine telle que la titin ou un de ses fragments sur la base de ses connaissances générales. Par ailleurs, plusieurs anticorps commerciaux sont disponibles, notamment dirigés contre la partie N-Terminale de la titin. A titre illustratif, il est possible d'utiliser l'IgG monoclonal de souris anti Titin (#H00007273-M07 clone 2F12, de chez Ab-nova) utilisé dans les exemples ci-dessous. Il est également possible d'utiliser l'IgG monoclonal de lapin anti titin dilué utilisé dans les exemples ci-dessous dirigé contre la partie C-ter (reconnaissant la séquence : NEFGSDSATVNINIRSMC (SEQ ID NO:46) ; acides aminés 35197 à 35213 chez la souris correspondant aux acides aminés : 34334 à 34349 chez l'être humain).

De même, la technique de spectrométrie de masse est bien connue de l'homme du métier et consiste en l'identification des protéines par la mesure des rapports *masse* sur *charge* (m/z) des peptides issus de digestion enzymatique (MS) ou de leurs fragments (MS/MS). Les masses des peptides générés sont ensuite comparées à celles des séquences protéiques présentes dans les bases de données permettant l'identification de la protéine. L'identification d'un ou plusieurs peptides de la titin, et plus particulièrement un peptide issu d'un fragment N-terminal ou C-terminal de la titin, comprenant notamment environ les 300 acides aminés N-terminaux ou les 150 acides aminés C-terminaux de la titin peut être utilisé dans le cadre de la présente invention. Selon un mode particulier de réalisation, le mode MRM (multiple reaction monitoring) est utilisé pour permettre la détection quantitative et/ou qualitative des peptides à travers toute la protéine.

Selon un autre aspect, la présente divulgation concerne un procédé de suivi d'une dystrophie musculaire tel que décrit ci-dessus. Dans ce cas, le procédé comprend la quantification du niveau de titin, ou d'un ou plusieurs fragments de titin à un temps T1 chez le sujet étudié, l'expression supérieure de ladite titin, dudit ou desdits fragments de titin à un temps T2 suivant T1 étant indicative d'une progression de la dystrophie et la même expression ou l'expression inférieure de ladite titin, dudit ou desdits fragments de titin à un temps T2 suivant T1 étant respectivement indicative d'une stabilisation ou d'une rémission. Cette évolution du niveau de la titin peut être aussi comparée à un échantillon standard d'un patient d'âge correspondant atteint d'une dystrophie musculaire.

Selon un autre aspect, l'invention concerne un procédé de suivi de l'efficacité d'un traitement d'une dystrophie musculaire. Dans ce cas, le procédé comprend la quantification du niveau de titin, d'un fragment ou des fragments de titin à un temps T1 chez le sujet étudié, l'expression inférieure de ladite titin, dudit ou desdits fragments de titin à un temps T2 suivant T1 étant indicative d'un traitement efficace de la dystrophie. Alternativement, le niveau de la titin ou d'un ou plusieurs de ses fragments à un temps T est comparé au niveau détecté dans un échantillon standard d'un patient atteint d'une dystrophie musculaire. Un niveau inférieur chez le sujet étudié sera indicatif d'un traitement efficace de la dystrophie.

Par "niveau d'expression supérieur" ou "niveau d'expression inférieur", on entend un niveau d'expression dont la variation est significative, selon des procédures bien connues de l'homme du métier.

Bien entendu, dans les deux procédés de suivi décrits ci-dessus, une méthode de dosage de la titin ou d'un ou plusieurs fragments de celle-ci sera employée, notamment un dosage par ELISA ou par spectrométrie de masse MRM

Selon les procédés de suivi de l'évolution d'une dystrophie et de suivi de l'efficacité d'un traitement, le premier échantillon et le second échantillon de liquide corporel sont prélevés de manière décalée dans le temps (par exemple plusieurs jours/semaines/mois après administration du traitement thérapeutique). Par ailleurs, dans le cas du suivi de l'efficacité d'un traitement, les premier et second échantillons peuvent être prélevés tous les deux après administration du traitement thérapeutique (par exemple, le premier échantillon est prélevé après traitement, le même jour que ce traitement, ou plusieurs jours/semaines/mois après le traitement, et le second échantillon est prélevé plusieurs jours/semaines/mois après le premier échantillon).

Le diagnostic, le suivi de l'évolution de la maladie ou l'efficacité du traitement pourra par ailleurs être confirmé dans des procédures suivant les procédés décrits dans la présente demande, comprenant des étapes connues d'évaluation d'une dystrophie musculaire (par exemple, détermination du niveau de créatine kinase, recherche de marqueurs spécifiques dans des biopsies musculaires, etc.)

L'invention concerne également une trousse de diagnostic d'une dystrophie musculaire, cette trousse comprenant les moyens de détection ou de dosage de la titin ou d'un fragment de cette dernière. Dans un mode particulier de réalisation, la trousse de diagnostic est une trousse comprenant les moyens de mise en oeuvre d'un test ELISA ou d'un Western-blot. A ce titre, la trousse diagnostic contient un moyen de détection de la titin ou d'un ou plusieurs fragment de cette dernière, notamment d'un fragment N-terminal ou C-terminal de la titin, le moyen de détection correspondant notamment à un ou plusieurs anticorps polyclonaux ou monoclonaux. La trousse peut également comprendre des instructions à suivre pour la mise en oeuvre du procédé selon l'invention.

La présente invention est illustrée par les figures et exemples suivants.

### Légende des figures

Figure 1. Identification d'un fragment N-terminal de la titin dans les urines DMD par électrophorèse bidimensionnelle.
Figure 2. Analyse de la présence du fragment N-terminal de la titin dans les urines de patients DMD et sains par Western blot.
Figure 3. Analyse de la présence du fragment N-terminal de la titin dans les urines d'autres dystrophies musculaires par Western blot.
Figure 4. Détection des peptides provenant des différentes parties de la titin par les approches « électrophorèse bidimensionnelle » et « LC-MS/MS » dans les urines de patients DMD.
Figure 5. Analyse de la présence du fragment N-terminal de la titin dans les urines de chiens GRMD et sains par Western blot.
Figure 6. Détection des peptides provenant des différentes parties de la titin par les approches « LC-MS/MS » dans les urines de patients DMD
Figure 7. Analyse de la présence du fragment N-terminal de la titin dans les urines de souris mdx et saines par Western blot
Figure 8. Analyse de la présence du fragment C-terminal de la titin dans les urines de patients DMD, de chiens GRMD et de souris mdx et de leurs contrôles sains respectifs par Western blot
Figure 9 Analyse par Western blot de la présence du fragment N-terminal ou C-terminal de la titin dans les urines et sérums de souris mdx et saines ayant subies un exercice physique
Figure 10 Identification, par l'approche « MALDI-TOF-TOF » d'un fragment intermédiaire de la titin dans les sérums de souris mdx ayant subies un exercice physique

### EXEMPLES

### Matériel et Méthodes :

### Analyse des protéines par l'Electrophorèse Bidimensionnelle :

Des protéines issues des urines de trois patients DMD et trois sujets sains ont été comparées par l'électrophorèse bidimensionnelle. L'isoélectrofocalisation est réalisée sur un gradient de pH immobilisé sur gel (IPG Strip pH 3-10, Biorad, dit « strip »). Une réhydratation passive du gel est effectuée en présence de l'échantillon solubilisé dans du tampon de dénaturation (7M Urée, 2M Thiouréc, 20mM DTT, 2% CHAPS, 1% ASB14, 1% Triton). La migration est effectuée selon le programme suivant : 50V pendant 5h, augmentation linéaire à 4000V durant 6h, puis 8000V pour un total de 30000Vh. Le strip est ensuite équilibré avec 3 ml de tampon (0,375M Tris-HCl pH 8,8 ; 6M Urée, 20% Glycérol, 2% SDS, 20mM DTT) pendant 10 minutes. L'étape d'équilibration est répétée 2 fois. Pour la 2e dimension, les strips sont transférés sur gel à gradient linéaire d'acrylamide (Precast Gel 4-20% Criterion TGX, Biorad). L'électrophorèse a été effectuée à 50V pendant 30 minutes puis 140V pendant lh30 dans du tampon TGS1x (25mM Tris, 192mM glycine, 0.1% SDS,). Les protéines sont ensuite colorées pendant 1 heure dans une solution de Bleu de Coomassie (Instant Blue, Expedeon) et décolorées par des bains successifs d'eau.

### Identification par spectrométrie de masse MALDI-TOF-TOF des protéines séparées par électrophorèse bidimensionnelle:

### - Digestion des protéines séparées par électrophorèse bidimensionnelle :

Les spots d'intérêt ont été extraits manuellement et placés dans des tubes Eppendorf 1,5 ml. Ils ont été lavés successivement avec 500 µl d'éthanol 10 minutes, 500µl d'eau 5 minutes et 500 µl d'éthanol 10 minutes. Après élimination de l'éthanol, la digestion a été effectuée sur la nuit à 37°C en présence de 30ng de trypsine porcine (Promega) par spot dans 10 µl de tampon NH4CO3 pH 7,9. Les peptides ont été dessalés/concentrés sur cônes ZipTip µC18 (Millipore) puis lavés avec 1% d'acide formique. Ils ont été ensuite élués dans 1µl de solution contenant 3mg/ml de matrice Acide α-cyano-4-hydroxycinnamique (HCCA), 80% d'acétonitrile et 1% d'acide formique afin d'être analysés par spectrométrie de masse MALDI-TOF-TOF.

### - Spectrométrie de masse, analyse MALDI-TOF-TOF :

Le système utilisé est un MALDI-TOF-TOF ABI 4800+ system (Applied Biosystems, Foster City, CA) couplé avec un laster 200Hz YAG (355nm). L'acquisition des spectres et le traitement des données ont été réalisées à l'aide du logiciel 4000 Series Explorer (version 3.5.1, Applied Biosystems). L'ensemble des spectres MS et MS/MS ont été soumis à un serveur Mascott « in house » (Matrix Science, Boston, MA) en utilisant la base de données Swissprot. La tolérance de la masse des ions fragments a été fixée à 100 ppm pour les ions précurseurs et de 0,3 Dalton pour les ions fragments.

### Identification des protéines totales par spectrométric de masse LC-MS/MS

### - Digestion des protéines en solution :

La totalité des protéines d'urine ou de sang (sérum) a subi une digestion enzymatique suivie d'une analyse LC-MS/MS. La digestion des protéines a été réalisée successivement avec deux enzymes : l'endoprotéinase Lys-C et la trypsine. Pour cela, 50 µg de protéines ont été solubilisés dans un volume de réaction de 50 µl contenant 50 mM Tris-Hcl pH 8,3 et 6M Urée et 2M Thiourée. Les protéines sont réduites avec 1 µl de DTT 500 mM (Concentration finale : 10 mM) à température ambiante pendant 30 minutes. Ensuite, les protéines sont alkylées avec 6 µl d'iodoacétamide 550 mM (Concentration finale : 55 mM) pendant 20 minutes. Les protéines sont digérées avec 5 µl d'endoprotéinase Lys-C (1 µg) pendant 3h à température ambiante à l'obscurité. Le mélange est dilué 4 fois avec 3 volumes d'eau mQ (195 µl pour un volume final de 260 µl ; Concentration finale en Urée/Thiourée 1,5 M/0,5 M) et les protéines sont digérées avec 10 µl de Trypsin (1 µg) pendant 16h à température ambiante et à l'obscurité. La digestion est terminée par l'ajout de 8,4 µl d'acide formique 100% (Concentration finale : 3%).

### - Spectrométrie de masse, analyse LC-MS/MS :

Les peptides sont analysés par chromatographie liquide couplée à un spectromètre de masse en tandem (LC-MS/MS). Le système utilisé est un spectromètre de masse LTQ Orbitrap Velos (Thermo Fisher Scientific, San Jose, CA) couplé à un système chromatographique Easy nano-LC Proxeon (Thermo Fisher Scientific, San Jose, CA). Les peptides en solution ont été dessalés/concentrés sur cônes ZipTip µC18 (Millipore) puis lavés avec 1% d'acide formique. Ensuite, ils ont été élués grâce à une solution à 50% acétonitrile 0.1% acide formique, séchés, puis repris de l'acide formique 0.1%. La séparation chromatographique des peptides a été réalisée avec les paramètres suivants: Colonne Proxeon Easy Column C18 (10 cm, 75 µm i.d, 120 A), avec un flux de 300 nl/min passant de 95% de solvant A (eau - 0,1% d'acide formique) à 25% de solvant B (100% d'acétonitrile, 0,1% d'acide formique) en 20 minutes, puis à 45% de B en 40 min et enfin à 80% de B en 10 min. Les peptides ont été analysés dans l'Orbitrap en mode balayage complet des ions avec une résolution de 30000 et une gamme de masses de 300 à 2000 m / z. Les fragments ont été obtenus par dissociation induite par collision (CID) avec une énergie d'activation collisionnelle de 40% et analysés dans le LTQ. Les données MS / MS ont été acquises dans un mode dans lequel les 20 ions précurseurs les plus intenses ont été isolés, avec une exclusion dynamique de 15 secondes. Les données ont été traitées avec le logiciel Proteome Discoverer 1.3 (Thermo Fisher Scientific, San Jose, CA) couplé à un serveur de recherche Mascot 'in house' (Matrix Science, Boston, MA; version 2.3.2). La tolérance de la masse des ions fragments a été fixée à 10 ppm pour les ions précurseurs et de 0,6 Dalton pour les fragments. L'oxydation (M) et la Carbamidométhylation (C) ont été considérées comme modifications variables possibles. Le nombre maximum d'erreur de clivage a été limité à deux pour la digestion par la trypsine. Les données MS-MS ont été analysées contre la base de données SwissProt

### Analyse des protéines par Western Blot :

Les échantillons d'intérêt ont été déposés sur gel 1D SDS-PAGE 4-12% Bis-tris (Invitrogen), la migration est effectuée pendant 30 minutes à 50 Volts puis 1 heure 40 à 140 Volts dans du tampon MOPSlx (Invitrogen). Ensuite, les protéines ont été transférées sur membrane PVDF (Millipore) pendant 2 heures à 40 Volts. La membrane est saturée dans du tampon Odyssey (LI-COR Biosciences) pendant la nuit à 4°C et incubé avec l'anticorps primaire pendant 1 heure sous agitation à température ambiante, suivie par 3 lavages au PBS-Tween 0,1% de 10 minutes chacun. La révélation a été réalisée grâce à l'incubation de la membrane avec un anticorps secondaire marqué par un fluorophore émettant dans l'infrarouge à 800 nm pendant 1 heure sous agitation à température ambiante à l'abri de la lumière suivie de 3 lavages PBS-Tween 0,1% de 10 minutes chacun puis de 2 lavages au PBS. Le signal fluorescent est révélé en scannant la membrane avec l'imageur Odyssey (LI-COR Biosciences).
Anticorps primaire (1) : IgG monoclonal de souris anti Titin à une concentration de 1µg/ml (#H00007273-M07 clone 2F12, de chez Ab-nova, dirigé contre la partie N-ter (acides aminés 1 à 111))
Anticorps primaire (2) : IgG monoclonal de lapin anti Titin dilué au 1/300^{e} dirigé contre la partie C-ter (séquence : NEFGSDSATVNINIRSMC ; acides aminés 35197 à 35213 chez la souris correspondant aux acides aminés : 34334 à 34349 chez l'humain) (Charton et al., 2010)
Anticorps secondaire (1) : Anticorps polyclonal de chèvre anti souris à une concentration de 0,1µg/ml (IRDye 800CW Goat anti-Mouse, LI-COR Biosciences)
Anticorps secondaire (2) : Anticorps polyclonal de chèvre anti lapin à une concentration de 0,1µg/ml (IRDye 800CW Goat anti-Rabbit, LI-COR Biosciences)

### Exercice physique chez la souris :

Deux groupes de 6 souris mâles (saines et mdx) ont été placés sur un tapis roulant incliné vers le bas (15°) afin d'effectuer une session de course de 30 minutes (8m/min pendant 5 minutes puis 12m/min pendant 25 minutes). Les urines et sérums des souris ont été collectés 7 jours avant exercice ainsi que 3h, 24h et 48h après exercice.

De plus, les souris mdx ne pouvant pas effectuer un exercice physique supérieur à 30 min, un groupe de 6 souris saines mâles a subi le même exercice mais durant lh30 (8m/min pendant 5 minutes puis 12m/min pendant lh25 minutes). Les urines et sérums des souris ont été collectés 7 jours avant exercice ainsi que 3h, 24h et 48h après exercice.

### Résultats :

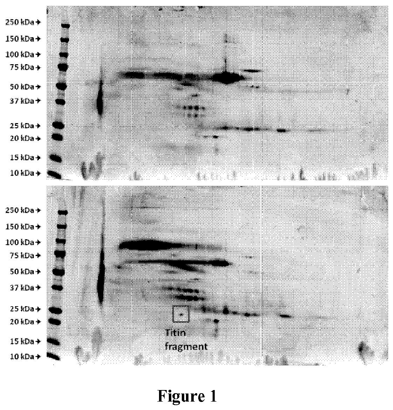

Les protéines ont été séparées comme décrit dans les Matériel et Méthodes. La figure 1 correspond à un gel représentatif de l'expérience. Le gel en haut présente les résultats de séparation des protéines d'un sujet sain, et le gel en bas - des protéines d'un individu DMD. Le spot encadré a été identifié par la spectrométrie de masse MALDI-ToF-ToF comme un fragment N-terminal de la titin. Ce fragment n'a été détecté que dans les urines de patients DMD. Les résultats sont représentatifs pour trois patients DMD et trois sujets sains différents.

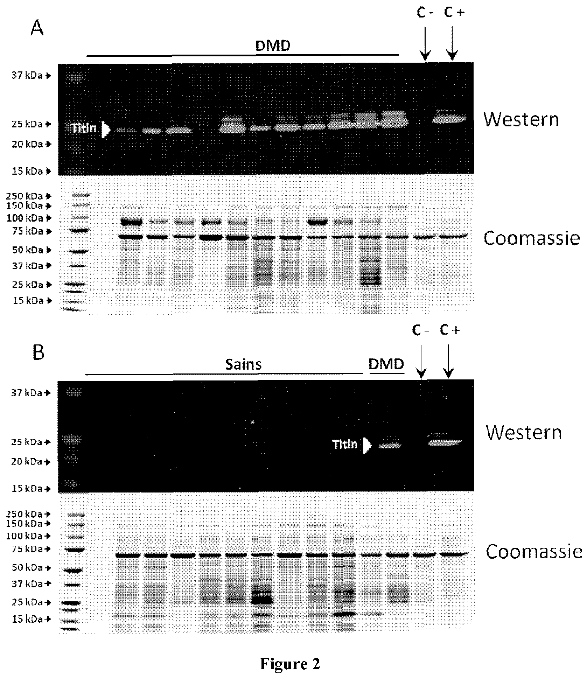

Les protéines ont été analysées par Western blot comme décrit dans les Matériel et Méthodes. La figure 2 correspond à un résultat représentatif dans lequel: le panneau (A) correspond aux résultats de l'analyse des patients DMD et (B) aux résultats des sujets sains. Le fragment N-terminal de la titin (signalé comme : « Titin » sur le Western) a été détecté dans 11 patients DMD sur 12, et il a été indétectable dans les 10 sujets sains analysés. C- contrôle négatif : urine du sujet sain précédemment analysé par électrophorèse tridimensionnelle ; C+ contrôle positif : urine du patient DMD analysé par électrophorèse bidimensionnelle.

Au total, 23 patients DMD ont été testés par cette méthode et le fragment N-terminal de la titin a été détecté dans 21 parmi eux. Ce fragment est absent des urines d'individus sains (13 sujets testés)

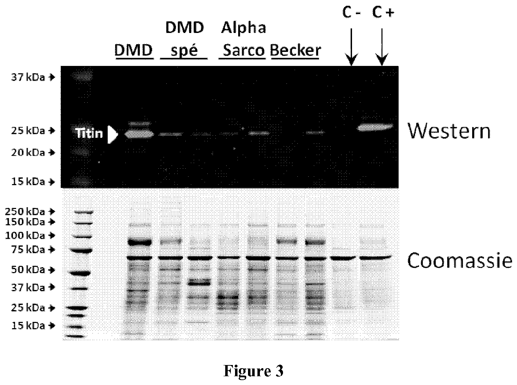

Les protéines ont été analysées par Western blot comme décrit dans les Matériel et Méthodes. La figure 3 présente les résultats d'analyse des urines de patients atteints des dystrophies musculaires suivantes: Dystrophie musculaire de Duchenne (DMD) ; Un cas particulier de DMD (patient qui marche à l'âge de 21 ans) (DMD spé) ; Alpha-sarcoglycanopathie (Alpha-Sarco) ; Dystrophie de Becker (Becker). Le fragment N-terminal de la titin (signalé comme : « Titin » sur le Western) a été détecté dans le cas particulier de DMD, chez les patients atteints de l'Alpha-sarcoglycanopathie (2 sur 2) et chez un patients atteint de la dystrophie de Becker (1 sur 2). C- contrôle négatif: échantillon d'urine du sujet sain analysé par électrophorèse bidimensionnelle; C+ contrôle positif: échantillon d'urine du patient DMD analysé par électrophorèse bidimensionnelle.

Les peptides provenant de la digestion des protéines d'urine ou de sang de patients DMD et sains ont été analysées par les approches « électrophorèse bidimensionnelle » et « LC-MS/MS » comme décrit dans les Matériel et Méthodes. Les peptides séquencés par MS/MS sont présentés dans la figure 4. Par l'approche « électrophorèse bidimensionnelle», les trois peptides appartenant à la partie N-terminale de la titin ont été identifiés chez les trois patients DMD. Un pool de 13 urines de patients DMD a aussi été analysé par l'approche « LC-MS/MS » (trois analyses différentes). Les mêmes peptides appartenant à la partie N-terminale de la titin ainsi que des peptides provenant d'autres parties de la titin, y compris la partie C-terminale (après l'acide aminé 34253) ont été identifiés dans ces analyses. Aucun peptide de la titin n'a été identifié dans les urines de sujets sains. Ces résultats montrent que d'autres parties de la titin peuvent être utilisées comme biomarqueurs. L'analyse du sang (sérum) de 4 patients DMD par l'approche « LC-MS/MS » a montré la présence du peptide 82-98 dans le sérum d'un seul patient. Aucun peptide de la titin n'a été identifié dans les sérums de 6 sujets sains testés par cette même approche (données non montrées).

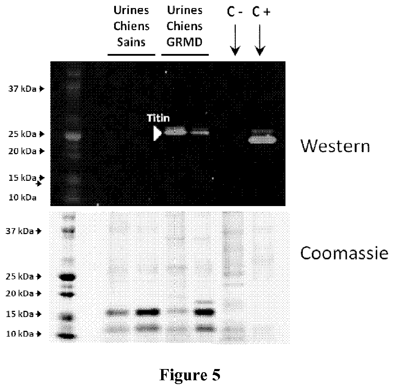

Les protéines ont été analysées par Western blot comme décrit dans les Matériel et Méthodes. Le fragment N-terminal de la titin (signalé comme : « Titin » sur le Western) a été détecté dans 2 chiens GRMD sur 2, et il a été indétectable dans les 2 chiens sains analysés (Figure 5). C- contrôle négatif: échantillon d'urine du sujet sain analysé par électrophorèse bidimensionnelle; C+ contrôle positif: échantillon d'urine du patient DMD analysé par électrophorèse bidimensionnelle.

### Etudes complémentaires :

Les peptides provenant de la digestion des protéines d'urine de patients DMD et sains ont été analysés par l'approche « LC-MS/MS » comme décrit dans les Matériel et Méthodes.
Les urines de 5 individus sains et 5 patients DMD ont été analysées individuellement par l'approche « LC-MS/MS ». L'analyse individuelle a permis de détecter plus de peptides de titin que dans l'analyse par pool décrite précédemment et a aussi montré qu'une majorité des peptides identifiés correspondaient aux parties N-terminale et C-terminale de la titin. Les peptides séquencés par MS/MS sont présentés dans la figure 6 avec le score d'identification pour chaque patient DMD.

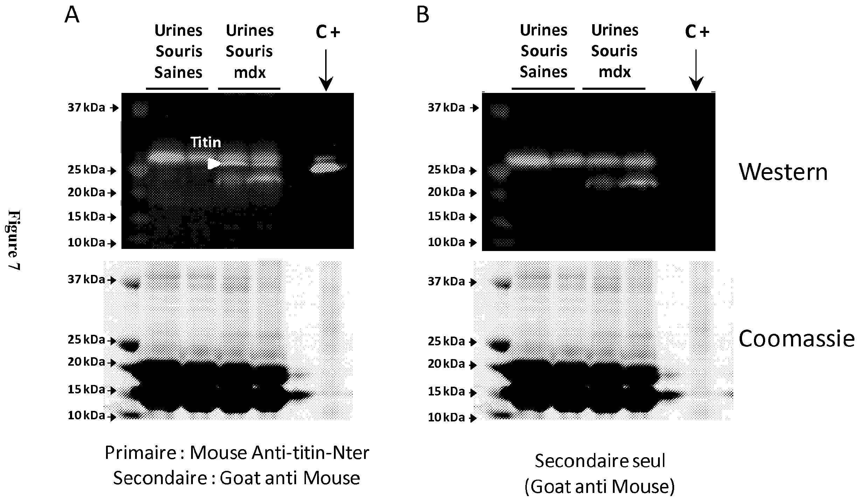

Les protéines ont été analysées par Western blot comme décrit dans les Matériel et Méthodes. Le fragment N-terminal de la titin (signalé comme : « Titin » sur le Western) a été détecté dans 2 souris mdx sur 2, et il a été indétectable dans les 2 souris saines analysées (Figure 7, A). Plusieurs bandes non spécifiques peuvent être observées et sont liées à l'anticorps secondaire (Figure 7, B). C+ contrôle positif: échantillon d'urine du patient DMD analysé par électrophorèse bidimensionnelle.

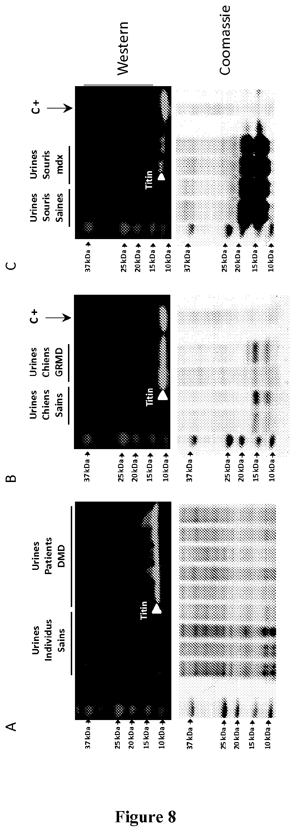

Les protéines ont été analysées par Western blot comme décrit dans les Matériel et Méthodes. Le fragment C-terminal de la titin (signalé comme : « Titin » sur le Western) a été détecté dans 5 patients DMD sur 5, et il a été indétectable dans les 4 sujets sains analysés. (Figure 8, A). Le fragment C-terminal de la titin (signalé comme : « Titin » sur le Western) a été détecté dans 2 chiens sur 2, et il a été indétectable dans les 2 chiens sains analysés. (Figure 8, B). Le fragment C-terminal de la titin (signalé comme : « Titin » sur le Western) a été détecté dans 2 souris mdx sur 2, et il a été indétectable dans les 2 souris saines analysées. (Figure 8, C). C+ contrôle positif: échantillon d'urine du patient DMD analysé par électrophorèse bidimensionnelle

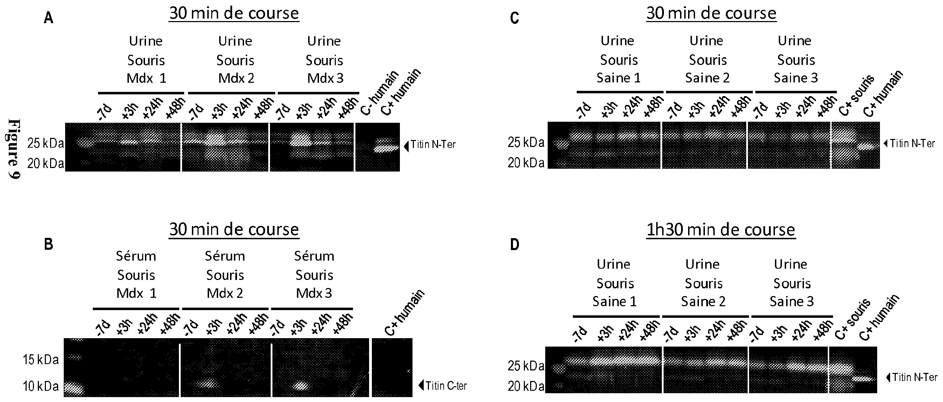

Les protéines ont été analysées par Western blot comme décrit dans les Matériel et Méthodes. Le fragment N-terminal de la titin (signalé comme : « Titin N-Ter » sur le Western) a été détecté dans toutes les souris mdx ayant subi un exercice physique avec une augmentation 3h après exercice (Figure 9, A). De plus, le fragment C-terminal de la titin a été détecté dans le sérum des souris mdx uniquement 3h après exercice. Ce fragment n'est pas détectable ni dans le contrôle humain, ni dans les sérums de souris n'ayant pas subi d'exercice (Figure 9, B) (Remarque : La recherche du fragment N-terminal de la titin dans le sérum des souris mdx est rendue impossible du fait de la présence sur le marché d'un seul anticorps primaire (souris anti-titin N-Ter) et donc de l'utilisation obligatoire d'un anticorps secondaire anti-souris sur du sérum de souris). De plus, le fragment N-terminal de la titin n'est toujours pas détectable dans les urines de souris saines ayant subi un exercice physique de 30 min ou même d'lh30 min (Figure 9, C et D). C- humain : contrôle négatif humain : échantillon d'urine d'un sujet sain; C+ humain contrôle positif humain : échantillon d'urine ou de sérum de patient DMD, C+ souris contrôle positif souris : échantillon d'urine de souris mdx.

Les protéines de sérums de souris mdx ayant subi un exercice physique de 30 min ont été séparées sur gel 1D SDS-PAGE comme décrit dans les Matériel et Méthodes. Les bandes d'intérêt (bandes encadrées sur la figure 10, A) ont été extraites manuellement et ont été identifiées par la spectrométrie de masse MALDI-ToF-ToF comme un fragment intermédiaire de la titin (de l'acide aminé : 12994 à 16442; qui correspond aux acides aminés 12132 à 15580 de la séquence humaine). Ce fragment n'a été détecté que dans les sérums de souris mdx ayant subi un exercice physique de 30 min et n'a pas été détecté dans les souris saines ayant subi un exercice physique de 30 min ou même d'lh30 min. (données non montrées). Les peptides séquencés par MALDI-TOF-TOF sont présentés dans la figure 10, B avec le score d'identification pour chaque souris mdx. De plus, la taille de ce fragment intermédiaire (∼3000 acides aminés, soit ∼300 kDa) identifiées par spectrométrie de masse MALDI-TOF-TOF correspond à la taille observée sur les gels SDS-PAGE (∼300 kDa).

En résumé, toutes ces expériences montrent que plusieurs fragments de titin (par exemple : N-terminal, C-terminal ou « intermédiaire ») sont détectés à la fois dans le sérum et l'urine de patients DMD, chiens GRMD et souris mdx et qu'ils ne sont pas détectés dans le sérum ou l'urine de sujets sains, chiens sains et souris saines.

### REFERENCES

Batchelor, C. L. and S. J. Winder (2006). "Sparks, signals and shock absorbers: how dystrophin loss causes muscular dystrophy." Trends Cell Biol 16(4): 198-205.
Bushby, K., R. Finkel, et al. "Diagnosis and management of Duchenne muscular dystrophy, part 1: diagnosis, and pharmacological and psychosocial management." Lancet Neurol 9(1): 77-93.
Charton, K, Danièle N, et al. (2010). "Removal of the calpain 3 protease reverses the myopathology in a mouse model for titinopathies." Hum Mol Genet 19(23): 4608-24
Cirak, S., V. Arechavala-Gomeza, et al. "Exon skipping and dystrophin restoration in patients with Duchenne muscular dystrophy after systemic phosphorodiamidate morpholino oligomer treatment: an open-label, phase 2, dose-escalation study." Lancet.
Le Rumeur, E., S. J. Winder, et al. "Dystrophin: more than just the sum of its parts." Biochim Biophys Acta 1804(9): 1713-22.
Lu, Q. L., T. Yokota, et al. "The status of exon skipping as a therapeutic approach to duchenne muscular dystrophy." Mol Ther 19(1): 9-15.
Matsumura et al., "Immunochemical study of connectin (titin) in neuromuscular diseases using a monoclonal antibody: connectin is degraded extensively in Duchenne muscular dystrophy", Journal of Neurological Sciences, vol. 93, no. 2-3, 1 novembre 1989, p. 147-156. Muntoni, F., S. Torelli, et al. (2003). "Dystrophin and mutations: one gene, several proteins, multiple phenotypes." Lancet Neurol 2(12): 731-40.
Nicholson, G. A., G. J. Morgan, et al. (1986). "The effect of aerobic exercise on serum creatine kinase activities." Muscle Nerve 9(9): 820-4.
Vassiliadis et al., "Clinical evaluation of a matrix metalloproteinase-12 cleaved fragment of titin as a cardiovascular serological biomarker", Journal of Translational Medicine, vol. 10, no. 1, 6 juillet 2012, p. 140.

## Revendications

1. Procédé pour le diagnostic d'une dystrophie musculaire chez un sujet, comprenant la détection dans un échantillon d'urine dudit sujet de la quantité de titin, ou de la quantité d'un ou plusieurs fragments de la titin, un niveau plus élevé de titin ou d'un ou plusieurs fragments de la titin dans l'échantillon prélevé du sujet par rapport à un sujet sain étant indicatif d'une dystrophie musculaire.

2. Procédé destiné à déterminer l'efficacité d'un traitement thérapeutique d'une dystrophie musculaire chez un sujet, comprenant
a) la mesure du niveau d'expression dans un échantillon d'urine de la titin, ou d'un ou plusieurs de ses fragments, moyennant quoi un niveau de référence est déterminé; puis
b) la mesure du niveau d'expression de ladite titin, dudit ou desdits fragments dans un second échantillon d'urine prélevé sur le même sujet à un temps donné après l'administration du traitement thérapeutique, moyennant quoi un niveau de test est déterminé; et
c) la comparaison des niveaux contrôle et de test, la diminution du niveau d'expression de la titin, d'un ou des fragments choisis étant indicative d'un traitement efficace de la dystrophie.

3. Procédé selon la revendication 1 ou 2, le fragment de la titin étant un fragment de la titin comprenant les acides aminés 42-62 (SEQ ID NO:12), 82-98 (SEQ ID NO:13), 99-109 (SEQ ID NO:14), 110-116 (SEQ ID NO:15), 127-136 (SEQ ID NO:16), 932-950 (SEQ ID NO:17), 1004-1012 (SEQ ID NO:18), 11784-11797 (SEQ ID NO:19), 14245-14257 (SEQ ID NO:20) 34253-34271 (SEQ ID NO:21), 34258-34271 (SEQ ID NO:22), 34272-34295 (SEQ ID NO:23), 185-202 (SEQ ID NO:24), 11958-11982 (SEQ ID NO:25), 16783-16809 (SEQ ID NO:26), 33320-33333 (SEQ ID NO:27), 34097-34111 (SEQ ID NO:28), 34272-34294 (SEQ ID NO:29) ou 34304-34322 (SEQ ID NO:30) de la titin.

4. Procédé selon la revendication 1 ou 2, le fragment de la titin étant un fragment de la titin murine comprenant les acides aminés 12994-13002 (SEQ ID NO:31), 13352-13362 (SEQ ID NO:32), 13542-13558 (SEQ ID NO:33), 14512-14524 (SEQ ID NO:34), 14867-14883 (SEQ ID NO:35), 14975-14993 (SEQ ID NO:36), 15034-15053 (SEQ ID NO:37), 15203-15214 (SEQ ID NO:38), 15234-15248 (SEQ ID NO:39), 15636-15645 (SEQ ID NO:40), 15746-15759 (SEQ ID NO:41), 15968-15987 (SEQ ID NO:42), 16034-16048 (SEQ ID NO:43), 16133-16148 (SEQ ID NO:44) et/ou 16432-16442 (SEQ ID NO:45) de la titin.

5. Procédé selon la revendication 1 ou 2, le fragment de la titin étant notamment un fragment N-terminal ou C-terminal de la titin.

6. Procédé selon l'une quelconque des revendications 1 ou 2, le fragment de la titin étant notamment un fragment N-terminal de la titin d'environ 300 acides aminés ou C-terminal de la titin d'environ 150 acides aminés.

7. Procédé selon l'une quelconque des revendications 1 à 6 pour le diagnostic de la dystrophie musculaire de Duchenne, de la dystrophie musculaire de Becker ou d'une alpha-sarcoglycanopathie.

8. Utilisation d'une trousse diagnostic comprenant les moyens de détection de la titin ou d'un ou plusieurs de ses fragments, pour la mise en oeuvre d'un procédé selon l'une quelconque des revendications 1 à 7.

## Patentansprüche

1. Verfahren zur Diagnose einer Muskeldystrophie bei einer Person, umfassend den Nachweis in einer Urinprobe besagter Person der Menge an Titin oder der Menge eines oder mehrerer Titin-Fragmente, wobei ein erhöhtes Niveau von Titin oder einem oder mehreren Titin-Fragmenten in einer von der Person erhaltenen Probe bezogen auf eine gesunde Person ein Hinweis auf eine Muskeldystrophie ist.

2. Verfahren zur Bestimmung der Wirksamkeit einer therapeutischen Behandlung einer Muskeldystrophie bei einer Person, umfassend
a) Messung des Expressionsniveaus von Titin oder einem oder mehreren seiner Fragmente in einer Urinprobe, wodurch ein Referenzniveau bestimmt wird; dann
b) Messung des Expressionsniveaus von besagtem Titin, besagtem oder besagten Fragmenten in einer zweiten Urinprobe, die von derselben Person zu einer gegebenen Zeit nach Verabreichung der therapeutischen Behandlung erhalten wird, wodurch ein Testniveau bestimmt wird; und
c) Vergleich von Kontrollniveau mit Testniveau, wobei die Verringerung des Expressionsniveaus von Titin, einem oder ausgewählten Fragmenten ein Hinweis auf eine wirksame Behandlung der Dystrophie ist.

3. Verfahren gemäß Anspruch 1 oder 2, wobei das Titin-Fragment ein Titin-Fragment ist, umfassend die Aminosäuren 42-62 (SEQ ID NO: 12), 82-98 (SEQ ID NO: 13), 99-109 (SEQ ID NO: 14), 110-116 (SEQ ID NO: 15), 127-136 (SEQ ID NO: 16), 932-950 (SEQ ID NO: 17), 1004-1012 (SEQ ID NO: 18), 11784-11797 (SEQ ID NO: 19), 14245-14257 (SEQ ID NO: 20), 34253-34271 (SEQ ID NO: 21), 34258-34271 (SEQ ID NO: 22), 34272-34295 (SEQ ID NO: 23), 185-202 (SEQ ID NO: 24), 11958-11982 (SEQ ID NO: 25), 16783-16809 (SEQ ID NO: 26), 33320-33333 (SEQ ID NO: 27), 34097-34111 (SEQ ID NO: 28), 34272-34294 (SEQ ID NO: 29) oder 34304-34322 (SEQ ID NO: 30) von Titin.

4. Verfahren gemäß Anspruch 1 oder 2, wobei das Titin-Fragment ein murines Titin-Fragment ist, umfassend die Aminosäuren 12994-13002 (SEQ ID NO: 31), 13352-13362 (SEQ ID NO: 32), 13542-13558 (SEQ ID NO: 33), 14512-14524 (SEQ ID NO: 34), 14867-14883 (SEQ ID NO: 35), 14975-14993 (SEQ ID NO: 36), 15034-15053 (SEQ ID NO: 37), 15203-15214 (SEQ ID NO: 38), 15234-15248 (SEQ ID NO: 39), 15636-15645 (SEQ ID NO: 40), 15746-15759 (SEQ ID NO: 41), 15968-15987 (SEQ ID NO: 42), 16034-16048 (SEQ ID NO: 43), 16133-16148 (SEQ ID NO: 44) und/oder 16432-16442 (SEQ ID NO: 45) von Titin.

5. Verfahren gemäß Anspruch 1 oder 2, wobei das Titin-Fragment insbesondere ein N-terminales oder ein C-terminales Titin-Fragment ist.

6. Verfahren gemäß einem der Ansprüche 1 oder 2, wobei das Titin-Fragment insbesondere ein N-terminales Titin-Fragment mit ungefähr 300 Aminosäuren oder ein C-terminales Titin-Fragment mit ungefähr 150 Aminosäuren ist.

7. Verfahren gemäß einem der Ansprüche 1 bis 6 für die Diagnose der Duchenne-Muskeldystrophie, der Becker-Muskeldystrophie oder einer alpha-Sarcoglykanopathie.

8. Verwendung eines Diagnosekits, umfassend die Mittel zur Detektion von Titin oder einem oder mehreren seiner Fragmente, für die Durchführung eines Verfahrens gemäß einem der Ansprüche 1 bis 7.

## Claims

1. Process for the diagnosis of muscular dystrophy in a subject, comprising detecting in a urine sample of said subject the amount of titin, or the amount of one or more fragments of titin, a higher level of titin or of one or more fragments of titin in the sample taken from the subject in comparison to a healthy subject being indicative of muscular dystrophy.

2. Process for determining the efficacy of a therapeutic treatment for muscular dystrophy in a subject, comprising
(a) measuring the level of expression of titin or of one or more of its fragments in a urine sample, by means of which a reference level is determined; then
(b) measuring the level of expression of said titin or of said fragment(s) in a second urine sample taken from the same subject at a given time after administering the therapeutic treatment, by means of which a test level is determined; and
(c) comparing the control level and the test level, the reduction of the level of expression of titin or of one or more selected fragments being indicative of an effective treatment of the dystrophy.

3. Process according to claim 1 or 2, the titin fragment being a titin fragment comprising amino acids 42-62 (SEQ ID NO: 12), 82-98 (SEQ ID NO: 13), 99-109 (SEQ ID NO: 14), 110-116 (SEQ ID NO: 15), 127-136 (SEQ ID NO: 16), 932-950 (SEQ ID NO: 17), 1004-1012 (SEQ ID NO: 18), 11784-11797 (SEQ ID NO: 19), 14245-14257 (SEQ ID NO: 20) 34253-34271 (SEQ ID NO: 21), 34258-34271 (SEQ ID NO: 22), 34272-34295 (SEQ ID NO: 23), 185-202 (SEQ ID NO: 24), 11958-11982 (SEQ ID NO: 25), 16783-16809 (SEQ ID NO: 26), 33320-33333 (SEQ ID NO: 27), 34097-34111 (SEQ ID NO: 28), 34272-34294 (SEQ ID NO: 29) or 34304-34322 (SEQ ID NO: 30) of titin.

4. Process according to claim 1 or 2, the titin fragment being a fragment of mouse titin comprising amino acids 12994-13002 (SEQ ID NO: 31), 13352-13362 (SEQ ID NO: 32), 13542-13558 (SEQ ID NO: 33), 14512-14524 (SEQ ID NO: 34), 14867-14883 (SEQ ID NO: 35), 14975-14993 (SEQ ID NO: 36), 15034-15053 (SEQ ID NO: 37), 15203-15214 (SEQ ID NO: 38), 15234-15248 (SEQ ID NO: 39), 15636-15645 (SEQ ID NO: 40), 15746-15759 (SEQ ID NO: 41), 15968-15987 (SEQ ID NO: 42), 16034-16048 (SEQ ID NO: 43), 16133-16148 (SEQ ID NO: 44) and/or 16432-16442 (SEQ ID NO: 45) of titin.

5. Process according to claim 1 or 2, the titin fragment being an N-terminal or C-terminal titin fragment.

6. Process according to any one of claims 1 or 2, the titin fragment being an N-terminal titin fragment of approximately 300 amino acids or a C-terminal titin fragment of approximately 150 amino acids.

7. Process according to any one of claims 1 to 6 for the diagnosis of Duchenne muscular dystrophy, Becker muscular dystrophy or alpha-sarcoglycanopathy.

8. Use of a diagnostic kit comprising means for detecting titin or one or more fragments thereof, for implementing a process according to any one of claims 1 to 7.
